(19) Europäisches Patentamt
European Patent Office
Office européen des brevets

(11) Publication number: 0 022 440 A1

# (12) EUROPEAN PATENT APPLICATION

(21) Application number: 79301217.0

(22) Date of filing: 22.06.79

(51) Int. Cl.³: C 07 C 67/31, C 07 C 69/40, C 07 C 69/716, C 07 C 69/757, A 61 K 31/215, A 61 K 31/22

(43) Date of publication of application: 21.01.81 Bulletin 81/3

(84) Designated Contracting States: CH DE FR GB

(71) Applicant: THE UPJOHN COMPANY, 301 Henrietta Street, Kalamazoo, Michigan 49001 (US)

(72) Inventor: Hessler, Edward James, 2503 Lomond, Kalamazoo, Michigan (US)
Inventor: Amin, Sanjay Indubhai, Race Course Circle, Baroda, Gujarat (IN)

(74) Representative: Perry, Robert Edward et al, GILL JENNINGS & EVERY 53-64 Chancery Lane, London WC2A 1HN (GB)

(54) **Process for preparing Michael addition products and for converting them to ibuprofen, an anti-inflammatory compound.**

(57) A Michael condensation product is prepared from $\alpha$-acetyl-$\alpha'$-methylsuccinate esters and isobutyl vinyl ketone using a dipolar, aprotic liquid solvent, preferably a dialkyl sulfoxide, a dialkylformamide or a dialkylacetamide, and potassium tert-butoxide. The Michael product of this process can be converted to ibuprofen, an anti-inflammatory drug.

EP 0 022 440 A1

TITLE MODIFIED
see front page



The Upjohn Company
GJE 679/168

# PROCESS FOR PREPARING MICHAEL ADDITION PRODUCTS AND CONVERTING THEM TO AN ANTI-INFLAMMATORY COMPOUND

This invention relates to processes for preparing ibuprofen, a known anti-inflammatory drug, via a process which produces one or more Michael condensation products, each of which can be converted to ibuprofen.

Ibuprofen, i.e. 2-(4-isobutylphenyl)propionic acid, is useful in the treatment of rheumatoid arthritis, among other diseases. It has fewer side effects than aspirin. The production of ibuprofen has hitherto usually been carried out from aromatic starting materials. Recently, it has been proposed to prepare ibuprofen from aliphatic starting materials, namely by reacting acetoacetate esters with α-halopropionate esters to form α-acetyl-α'-methylsuccinate diesters, and reacting these succinate diesters with isobutyl vinyl ketone, or its Mannich base equivalent, to form a Michael condensation product. For example, British Patent Application No. 8271/78 discloses the use of a deprotonating base (e.g., potassium carbonate), of a particular particle size, as a condensing agent and a nonpolar aprotic organic liquid, e.g., toluene, as solvent. Thereafter, the Michael condensation product is converted ("aromatised") to ibuprofen. One such process for aromatising the Michael condensation product is described in British Patent Application No. 20424/77 and involves heating the Michael condensation product with a sulfonic or phosphonic acid at from 75 to 130$^{\circ}$C in a nonpolar organic liquid which

azeotropes with water. Ibuprofen and ibuprofen esters are produced. The esters can be converted to ibuprofen by known procedures.

This invention concerns an improvement on the first of the two steps described above.

According to the present invention, a process for preparing a Michael addition product comprises reacting a di($C_{1-4}$ alkyl) α-acetyl-α'-methylsuccinate ester and isobutyl vinyl ketone at from 10 to 25°C in the presence of (i) 4 to 50%, by volume based on the total reaction mixture, of a dipolar aprotic liquid solvent and (ii) potassium <u>tert</u>-butoxide. By using the specified solvent and base, the final yield of usable Michael reaction product are increased and the yield of ibuprofen, relative to previously proposed procedures, therefrom can be increased from about 45 percent to about 69 percent, based on the α-acetyl-α'-methylsuccinate diester.

The Michael reaction product may comprise one or more of the compounds of formulae I, II, III and IV.

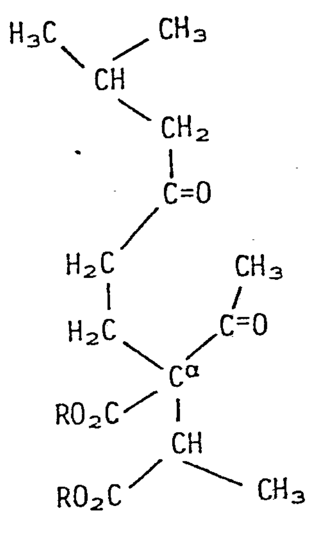

I

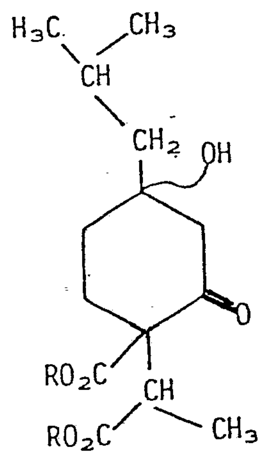

II

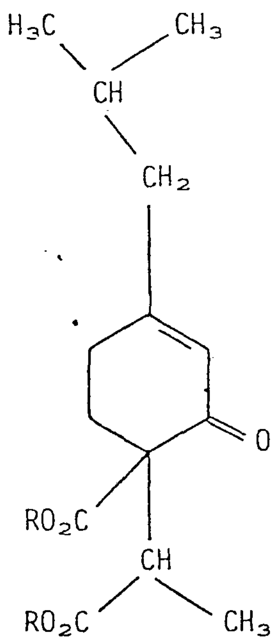

III

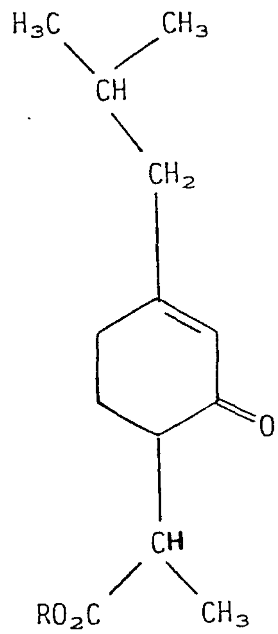

IV

and polymers thereof, where each R is $C_{1-4}$ alkyl. The succinate ester is preferably the diethyl ester. Therefore, R is preferably ethyl.

Solvents which may be used, other than the dipolar, aprotic liquid solvent in the mixture, are small amounts of non-polar, aromatic liquid solvents for the succinate reagent. Examples of such non-polar solvents which can be used include toluene, benzene, xylene and chlorobenzenes. Toluene is preferred. The reaction mixture may also contain any solvent used to carry and dilute the isobutyl vinyl ketone reactant, e.g. methyl isobutyl ketone.

The dipolar, aprotic liquid solvent is preferably a di($C_{1-4}$ alkyl)sulfoxide, e.g., dimethyl sulfoxide (DMSO), diethyl sulfoxide, dipropyl sulfoxide or dibutyl sulfoxide; a di($C_{1-4}$ alkyl)formamide, e.g., dimethylformamide (DMF), diethylformamide, dipropylformamide or dibutylformamide; a di($C_{1-4}$ alkyl)acetamide, e.g., dimethylacetamide (DMAC), diethylacetamide, dipropylacetamide or

dibutylacetamide. Prepared or commercial mixtures of these solvents may be used. DMSO is a very good choice if only this process step is being considered. DMF is usually preferred since it is often a better choice where the efficiency of solvent recovery is considered with the operation of preceding and/or subsequent steps in the overall synthesis of ibuprofen.

It is particularly preferred that DMF, or another dipolar, aprotic solvent, is used in the reaction of the preceding step when an α-halopropionate ester is reacted with an acetoacetate ester to form the α-acetyl-α'-methylsuccinate ester starting material for use in the process of this invention. It has been found that the use of DMF in particular mole ratios in that ester/ester alkylation step increases the dielectric constant of the mixture and eliminates the need to control the particle size of the deprotonating base used therein. That discovery now provides the additional advantage that only two solvents (toluene and DMF) instead of three solvents (toluene, DMF and DMSO need be handled and recovered in the operation of two process steps; (a) α-halopropionate ester plus acetoacetate ester to form the α-acetyl-α'-methylsuccinate ester and (b) isobutyl vinyl ketone plus α-acetyl-α'-methylsuccinate ester.

Other dipolar, aprotic solvents can be used in the invention, particularly those which have dielectric constants above about 25 at 25°C and which form complexes with metal ions, e.g., the potassium from the potassium <u>tert</u>-butoxide. The solvents described above, however, are the most practical, readily available, and preferred dipolar, aprotic solvents.

The reaction temperature is preferably from 15 to 20°C.

To begin the reaction, it is convenient to mix the crude dialkyl α-acetyl-α'-methylsuccinate ester with the selected dipolar, aprotic solvent, preferably DMF, so that the mixture is about 50:50 ester: solvent by volume, then to add the potassium <u>tert</u>-butoxide, and subsequently

to begin adding the isobutyl vinyl ketone reactant, e.g., in methyl isobutyl ketone solution, e.g., over about 4.5 hours. At the end of this period, the content of dipolar, aprotic organic liquid solvent in the mixture may be as low as 4% by volume. The reaction to form the Michael addition product can be considered essentially complete after the addition of the ketone, but it may be convenient, on occasion, to stir the reaction mixture in order to ensure complete reaction. This can be done without harmful effects on the yields of the Michael adduct product or the ibuprofen obtained therefrom. A reaction which is at about 93% conversion to Michael condensation product, within one to two hours after the last isobutyl vinyl ketone addition, can be at about 94 to 95% conversion if the mixture is stirred overnight.

Potassium tert-butoxide is the base for use in this invention. Other basic catalysts are operative, but process runs using sodium or potassium methoxides or ethoxides in these reaction mixtures produced lower yields.

The following Examples illustrate the invention.

EXAMPLE 1

A mixture of 31.4 ml. (8mmol.) of diethyl α-acetyl-α'-methylsuccinate, 25 ml. of dimethyl sulfoxide, and 6 ml. of a 20% solution of potassium t-butoxide in tetrahydrofuran are introduced into a round-bottom flask under a nitrogen atmosphere. The mixture is cooled to 16-18°C. A mixture of 23% vinyl isobutyl ketone/77% methyl isobutyl ketone is added (under stirring) over 4.5 hours to the reactor while maintaining the temperature at 16-18°C. The total amount of vinyl isobutyl ketone added (in solution of methyl isobutyl ketone) is 62.4 g. (128 mmol.).

After the addition of vinyl isobutyl ketone is completed, 90 ml. of methyl isobutyl ketone and 50 ml. of water added and the phases are allowed to separate. The aqueous phase is discarded. The organic phase is washed with 50 ml. of water, dried over anhydrous sodium sulfate and distilled at 60°C and 4 mm. Hg. to eliminate

the excess vinyl isobutyl ketone. 45.2 g. of a mixture of Michael products (formulae I to IV, and polymers thereof, in which R is ethyl) are obtained (90% yield).

EXAMPLE 2

A mixture of 45.2 g. of the Michael reaction product of Example 1 and 27.4 g. of p-toluenesulfonic acid monohydrate is heated in an oil bath to about 80°C for two hours. The mixture is further heated to 116°C for 8.5 hours, while removing the by-products water and ethanol. The reaction mixture is cooled to 90°C and toluene and water are added to precipitate p-toluenesulfonic acid monohydrate which is then filtered off. The toluene filtrate, containing ibuprofen, is treated with 4.5 g. of sodium hydroxide in water to extract out the sodium salt of ibuprofen. 13 g. of sodium hydroxide and 75 ml. acetone are added to the aqueous mixture. The sodium salt of ibuprofen is crystallised by cooling to between 0 and 10°C and filtered. The sodium salt is dissolved in water, stirred with Skellysolve B ( a mixture of isomeric hexanes), and acidified with sulfuric acid to extract ibuprofen into the organic solvent phase.

Evaporation of the solvent from the organic phase gives 11.6 g. ibuprofen, a chemical yield of 78.2% based on the Michael product.

In other, later, syntheses, yields of ibuprofen up to 85%, based on the Michael reaction product, were obtained.

Comparative Example A

When the Michael reaction was run in the known manner, avoiding the presence of a dialkyl sulfoxide (DMSO) or a dialkylformamide (DMF), the final yields from succinate were only up to 45% even when the catalyst was potassium t-butoxide. This shows that a 60% yield gain can be achieved by using the process of this invention.

The following Examples illustrate the preparation of the succinate ester using DMF as solvent.

EXAMPLE 3

To a stirred slurry of 20.7 g. of potassium carbonate, milled to 200 mesh and dried overnight at 80°C/40 mm. Hg., 1.5 g. of milled potassium iodide and 1.5 g. of Aliquat 336 (believed to be tricaprylyl methylammonium chloride) in a solution of 25 ml. of DMF and 25 ml. of toluene, at room temperature, 14.5 ml. of ethyl 2-chloropropionate and 14.5 ml. of ethyl acetoacetate are added. The resulting slurry mixture is heated to 100 to 105°C over 30 minutes, stirred for another 90 minutes and then cooled to 17°C over the next 75 minutes, to form diethyl α-acetyl-α'-methylsuccinate. Then 110 ml. of two percent sulfuricacid is added slowly (some foaming) to lower the pH of the mixture to 8.5, and the phases are allowed to separate. The aqueous phase is backwashed with 20 ml. of toluene and the combined toluene phases are passed through 10 g. of anhydrous sodium sulfate. The filter cake is washed with about 50 ml. of toluene. The combined filtrates are mixed well and a sample thereof is submitted for analysis. Gas liquid chromatography analysis shows that the product mixture contains 190.4 mg. of diethyl α-acetyl-α'-methylsuccinate per ml. of sample. Using that figure to determine yield, 112 ml. of the above toluene filtrate mixture contains 21.33 g. of the succinate ester intermediate product for an 82.0% yield.

EXAMPLE 4

A slurry of 267 kg. calcined, granular $K_2CO_3$, 19.3 kg. each of KI and Aliquat 336, 311 Kg. of DMF, and 276 l. of toluene was stirred at reflux (118-120°C) for two hours while water was azeotroped off and collected in a trap. The resulting dried slurry was cooled to below 80°C. 189 kg. of ethyl acetoacetate and 198 kg. of ethyl 2-chloropropionate were then added and the reaction mixture was stirred at 103°C for 90 minutes.

120 kg. toluene were added and the reaction mixture was cooled to 20°C. The salts were filtered off using a centrifuge and the filtrate (toluene plus product) was washed three times with water (280 l., 140 l. and 140 l.). The combined aqueous washes were backwashed with 85 kg. toluene. The combined toluene phases were concentrated

under vacuum at 65°C to leave 348 kg. (350 l.) of crude succinate; assay 663 mg./ml. for a yield of 69% chemical.

The procedure as described above was repeated, except that the $K_2CO_3$ was milled to <200 mesh before use. The chemical yield with this modification was 85%.

CLAIMS

1. A process for preparing a Michael reaction product, which comprises reacting a di($C_{1-4}$ alkyl)α-acetyl-α'-methylsuccinate ester with isobutyl vinyl ketone at from 10 to 25°C in the presence of (i) 4 to 50%, by volume of the total mixture, of a dipolar, aprotic liquid solvent and (ii) potassium tert-butoxide.

2. A process according to claim 1 which is conducted at from 15 to 20°C.

3. A process according to claim 1 or claim 2 wherein the succinate ester is diethyl α-acetyl-α'-methylsuccinate.

4. A process according to any preceding claim wherein the dipolar aprotic liquid solvent is selected from di($C_{1-4}$ alkyl) sulfoxides, di($C_{1-4}$ alkyl)formamides and di-($C_{1-4}$ alkyl)acetamides.

5. A process according to claim 4 wherein the dipolar, aprotic liquid solvent is selected from dimethyl sulfoxide, dimethylformamide and dimethylacetamide.

6. A process according to claim 4 wherein the dipolar, aprotic liquid solvent is a ($C_{1-4}$ alkyl)formamide.

7. A process according to claim 6 wherein the dialkylformamide is dimethylformamide.

8. A process according to claim 6 or claim 7 wherein the succinate ester is prepared by reacting an α-halopropionate ester with an acetoacetate ester in the presence of a di($C_{1-4}$)alkylformamide and a deprotonating base.

9. A process according to any preceding claim wherein the Michael reaction product is converted to ibuprofen.

European Patent Office

# EUROPEAN SEARCH REPORT

0022440

Application number

EP 79 301 217.0

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | DE - A1 - 2 452 459 (AZIENDE CHIMICHE RIUNITE ANGELINI FRANCESCO)<br>* examples 1, 6 and 7 *<br>--<br>DE - A1 - 2 806 424 (UPJOHN)<br>* claims 5 to 8 and 10 *<br>---- | 1,8,<br>9<br><br><br>1,8,<br>9 | C 07 C 67/31<br>C 07 C 69/40<br>C 07 C 69/716<br>C 07 C 69/757<br>A 61 K 31/215<br>A 61 K 31/22 |
| | | | **TECHNICAL FIELDS SEARCHED (Int.Cl.3)** |
| | | | A 61 K 31/215<br>A 61 K 31/22<br>C 07 C 67/31<br>C 07 C 69/40<br>C 07 C 69/716<br>C 07 C 69/757 |
| | | | **CATEGORY OF CITED DOCUMENTS** |
| | | | X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| Berlin | 24-01-1980 | KNAACK |

EPO Form 1503.1 06.78